# EUROPEAN PATENT APPLICATION

(11) **EP 2 368 885 A1**
(43) Date of publication of application: **28.09.2011**
(21) Application number: 10305284.1
(22) Date of filing: 23.03.2010
(51) Int. Cl.: C07D 241/46, A61P 35/00, A61K 31/498

(54) **Derivatives of phenazine useful to treat cancer**

(71) Applicant: Université de la Méditerranée (Aix-Marseille II), 13284 Marseille Cédex 07 (FR); Centre National de la Recherche Scientifique (C.N.R.S.), 75794 Paris Cedex 16 (FR)
(72) Inventor: The designation of the inventor has not yet been filed
(74) Representative: Le Coupanec, Pascale A.M.P.

(57) **Abstract**

The present invention relates to derivatives of phenazine having a general formula (I) as follows: 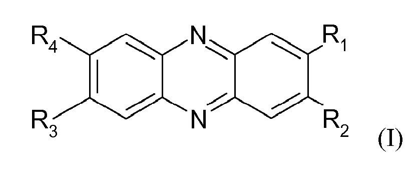 for use as an agent for treating cancer.

## Description

The present invention relates to derivatives of phenazine and to their application in therapeutics, and in particular to treat the cancer.

Mortality rate from cancer has considerably increased during the last decades. Particularly, the incidence of gastrointestinal cancers, such as pancreatic cancer, colon cancer or gastric cancer is high.

Concerning pancreatic cancer, it is ranked fifth among cancer deaths in men, and six among cancer deaths in women. Pancreatic cancer has poor prognosis, and the five-year survival rate of resected cases is 5 to 20 %. Poor prognosis of pancreatic cancer is due to the fact that, in many cases, even a small cancer lesion (about 2 cm) invades organs outside of the pancreas, and metastasizes to the liver.

Chemotherapeutic agent for the treatment of pancreatic cancer has already been reported, such as doxorubicine or Gemcitabine. However, doxorubicine, an antibiotic of anthracycline type, has serious side effects, notably cardiotoxic side effects. Gemcitabine, an analogue of the deoxycytidine, is known as having efficiency with respect to pancreatic cancer usually less than 5 % and further as also displaying severe side effects like myelosuppression and interstitial pneumonia.

Accordingly, the use of such chemotherapeutic agents required to be used in a sufficiently high amount to provide a cytotoxic effect but also in a sufficiently low amount to not induce said side effects.

Thus, currently, there is a lack of chemotherapeutic agents being competent in order to be used as anti-cancer and anti-tumorigenic compound, in particular to be used for the treatment of pancreatic cancer, and further being without side effects.

For obvious reasons, the development of new compounds with less or without side effects presents a considerable interest.

The inventors have considered extensive studies on the antitumor effect of phenazine derivatives and as result have found that some compounds are effective chemotherapeutic agents, in particular against cancerous pancreatic cells (MiaPaca cells). Accordingly, it can constitute new target for the development of novel therapeutic strategies against cancers or diseases linked to uncontrolled and/or abnormal growth of cells, and notably may serve as lead compound for a new generation of anti-cancer agents.

It has been found that derivatives of formula (I) as defined hereafter are able to interfere with uncontrolled and abnormal growth of cells, in particular cancerous cells, as illustrated in the experimental data herein after.

The present invention therefore relates to compounds of formula (I) as defined below for use as agents for treating cancer, especially pancreatic cancer.

The present invention further relates to some particular derivatives such as defined below.

The present invention relates to one of said compounds for use as a medicament and also provides pharmaceutical compositions comprising at least one of said compounds.

According to a first aspect, a subject-matter of the present invention relates to a compound of formula (I): 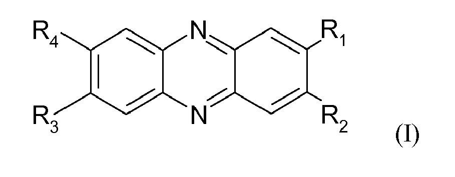 wherein R₁, R₂, R₃ and R₄, independently the ones of the others, represent:
- a hydrogen atom ;
- a halogen atom chosen among chlorine, fluorine, bromine and iodine atom;
- a cyano group ;
- -NO₂;
- -N(R₅)(R₆), with R₅ and R₆ may represent, independently the one of the other, a hydrogen atom, an alkyl group, linear or branched, in C₁-C₂₀, a benzyl group or - COR₁₀;
- an amido group ;
- -NHSO₂(R₇), with R₇ may represent an alkyl group, linear or branched, in C₁-C₂₀ or an aryl group;
- -OR₈;
- -OCOR₉, with R₈ and R₉ may represent a hydrogen atom or an alkyl group, linear or branched, in C₁-C₂₀;
- an alkyl or alkylthio group, linear or branched, saturated or non saturated, in C₁-C₂₀;
- -COR₁₀ with R₁₀ may represent a hydroxyl group or an alkoxy group, linear or branched, in C₁-C₂₀; and
- -CO-[NH-(CH₂)ₙ]ₘ-N(R₅)(R₆), -HN-CO-[(CH₂)ₙ-N(R₁₁)]ₚ-(CH₂)_{n'}-N(R₁₁)(R₆), or -O-CO-[(CH₂)ₙ-N(R₁₁)]ₙ-(CH₂)ₙ-N(R₁₁)(R₆), with
- R₅ and R₆ being are such as defined above,
- R₁₁ representing a hydrogen atom or a tert-butyloxycarbonyl group,
- p representing an integer ranging from 0 to 1, and
- n, n' and m, independently, representing an integer with n and n', independently, ranging from 2 to 6 and m ranging from 1 to 2, and, when m is 2, each n value may be identical or different one from another,
or anyone of its pharmaceutically acceptable salts,
provided that when R₁ and R₃ represent a hydroxyl group, then at least one of R₂ or R₄ is different from a hydrogen atom,
for use as an agent for treating cancer.

More particularly, the present invention relates to a compound of formula (I) for use as an antitumor agent for the treatment of gastrointestinal cancer, and in particular pancreatic cancer.

Some of derivatives of phenazine are already known. Various compounds having such phenazine structure have already been disclosed in Laursen et al. (chem. Rev., 2004, 104:1663), Beifuss et al. (Top. Curr. Chem., 2005, 77), Terech et al. (J. of Coll. and Inter. Sc., 2006:633), Llusar et al. (Zeit. fuer Anorg. und Allge. Chem., 2005, 631:2215; J. of Mat. Chem., 2003, 13: 2505), Pozzo et al. (Mol. Crystals and Liquid Crystals Sc. and Tech., 2000, 344:101; J. of Mat. Chem, 1998, 8:2575) and US 2004/065227. However, to the knowledge of the inventors, these compounds have never been proposed as chemotherapeutic agents.

Only 2,7-dihydroxyphenazine has been described for various application, such as antibiotics, pesticides, anti-fungals, and anti-cancer (Endo et al., Sci. Rep. Res. Inst. Tohoku Univ., 1969: 16(1-2)).

Unexpectedly, the inventors identified that compounds according to formula (I) possess an effective anticancer power with, at the same time, a low toxicity with respect to the individual in need thereof, that is to say individual having a cancer, and in particular a pancreatic cancer.

As used herein, the term "cancer" means the uncontrolled, abnormal growth of cells and includes within its scope all the well known diseases that are caused by the uncontrolled and abnormal growth of cells. Non-limiting examples of common cancers include bladder cancer, breast cancer, ovarian cancer and gastric cancer, cervical cancer, colon cancer, endometrial cancer, head and neck cancer, lung cancer, melanoma, multiple myeloma, leukemia (e.g. myeloid, lymphocytic, myelocytic and lymphoblastic leukemias), non-hodgkin's lymphoma, prostate cancer, rectal cancer, malignant melanomas, and in particular pancreatic cancer.

According to the present invention, the terms below have the following meanings:
The terms mentioned herein with characteristics such as for example C₁-C₂₀ can also be used with lower numbers of carbon atoms such as C₁-C₃ or C₁-C₇. If for example the term C₁-C₅ is used, it means that the corresponding hydrocarbon chain may comprise from 1 to 5 carbon atoms. If for example the term C₃-C₈ is used, it means that the corresponding hydrocarbon chain or cycle may comprise from 3 to 8 carbon atoms.
The term "halogen atom" corresponds to a fluorine, chlorine, bromine or iodine atom. Chlorine is preferred halogen atom in the framework of the present invention.
The term "alkyl" as used herein refers to a saturated, linear or branched aliphatic group. The following examples may be cited: methyl, ethyl, 1-propyl, 2-propyl, 1-butyl, 2-methyl-1-propyl (also named *i*-Bu), 2-butyl (also named *s*-Bu), 2-methyl-2-propyl (also named t-Bu), 1-pentyl (also named *n*-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, n-pentyl, n-hexyl, n-heptyl, *n*-octyl, *n*-nonyl, n-decyl, *n*-undecyl, *n*-dodecyl, *n*-tridecyl, *n*-tetradecyl, *n*-pentadecyl, *n*-hexadecyl, *n*-heptadecyl, *n*-octadecyl. Preferred alkyl according to the invention are methyl, ethyl, 1-propyl, 2-propyl, 1-butyl, 2-methyl-1-propyl (also named *i*-Bu), 2-butyl (also named *s*-Bu), 2-methyl-2-propyl (also named t-Bu), 1-pentyl (also named *n*-pentyl), 2-pentyl, 3-pentyl, 2-methyl-2-butyl, 3-methyl-2-butyl, 3-methyl-1-butyl, 2-methyl-1-butyl.
The term "alkoxy" corresponds to a -O-alkyl group, wherein the alkyl group is as defined above. The following examples may be cited: methoxy, ethoxy, propoxy, isopropoxy.
The term "aryl" as used herein means an aromatic mono- or poly-cyclic group. An example of monocyclic group may be phenyl.
Rings as defined above may be bonded through a carbon atom or a heteroatom, if any.
Regardless of bond indications, if a substituent is polyvalent (based on its position in the structure referred to), then any and all possible orientations of the substituent are intended.
The compounds of formula (I) may comprise an unsaturation site and thus may be in their tautomeric form. The instant invention also extends to the compounds of formula (I) in their tautomeric form.
The compounds of formula (I) can be provided in the form of a free base or in the form of addition salts with acids, which also form part of the invention.

These salts can be prepared with pharmaceutically acceptable acids, but salts with other acids, useful for example for the purification or for the isolation of the compounds of formula (I), also form part of the invention.

Among the compound of formula (I) defined above, the compounds for which the use is preferred may be given in details as follows.

A variant of a preferred compound of the invention may be represented by a compound of formula (I), wherein R₁=R₂ and/or R₃=R₄, with R₁ to R₄ being such as defined above, for use as an agent for treating cancer.

In another preferred variant, a compound of the invention may be represented by a compound of formula (I), wherein R₁=R₂ and represent -OR₈ or -OCOR₉, with R₈ and R₉ being such as defined above, for use as an agent for treating cancer.

In still yet another preferred embodiment, a compound of formula (I) may be defined as a compound of formula (Ia) as follows: 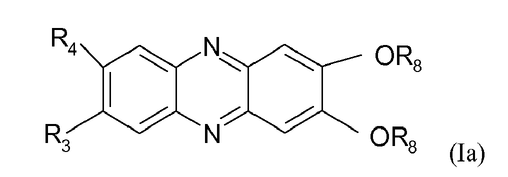 wherein R₃ and R₄ are such as defined above and R₈ represent an alkyl group, linear or branched, in C₁-C₂₀, or anyone of its pharmaceutically acceptable salts, for use as an agent for treating cancer.

A variant of a preferred compound of the invention may also be represented by a compound of formula (I) or (Ia), wherein R₃=R₄ and represent:
- a hydrogen atom;
- -N(R₅)(R₆);
- -NHSO₂(R₇); and
- a halogen atom, in particular chlorine,
with R₅, R₆ and R₇ being such as defined above, for use as an agent for treating cancer.

An other preferred variant of a compound of the invention may be represented by a compound of formula (I) or (Ia), wherein R₃=R₄ and represent a hydrogen atom or NH₂, for use as an agent for treating cancer.

Still according to a preferred variant, a compound of the invention may be represented by a compound of formula (Ia), wherein R₃=R₄ and represent a hydrogen atom or NH₂, with R₈ representing an alkyl group, linear or branched, in C₁-C₂₀, for use as an agent for treating cancer.

The present invention further relates to a compound of formula (I), wherein at least R₁, R₂ and R₃ are different from a hydrogen atom or to a compound of formula (Ia), wherein at least one of R₃ and R₄ is different from a hydrogen atom, for use as an agent for treating cancer.

Still according to a preferred variant, the present invention focuses on a compound of formula (I) or (Ia), wherein R₃ represents -COR₁₀, with R₁₀ being such as defined above, for use as an agent for treating cancer.

Among the preferred compounds according to the instant invention, the following list of compounds may be cited:
- 2,3-didodecanoxy-7-carboxyphenazine (compound 1) ;
- 2,3-didodecanoxy-7,8-diaminophenazine (compound 2) ;
- 2,3-didodecanoxyphenazine (compound 3) ;
- 2,3-didodecanoxy-7-dimethylaminoethylcarbamoylphenazine (compound 4) ;
- 2-hydroxy-3-ethoxy-7-ethylcarboxyphenazine (compound 5) ;
- phenazine (compound 6) ;
- 2,3-diopalmitoylphenazine (compound 7) ;
- 2,3-dihydroxy-7,8-dichlorophenazine (compound 8) ;
- 2,3-didodecanoxy-7,8-ditosylaminophenazine (compound 9) ;
- 2,3-diaminophenazine (compound 10) ;
- 2,3-dihydroxy-7-nitrophenazine (compound 11);
- 2,3-dihydroxy-7,8-ditosylaminophenazine (compound 12) ;
- 2,3-dihydroxy-7,8-diamino(Boc)phenazine (compound 13) ;
- 2,3-diethoxy-7-ethylcarboxyphenazine (compound 14) ;
- 2-hydroxyphenazine (compound 15) ;
- 2,3-dihydroxy-7-chlorophenazine (compound 16) ;
- 2,3-dihydroxy-7-carboxyphenazine (compound 17) ;
or anyone of its pharmaceutically acceptable salts,
for use as an agent for treating cancer.

More preferably, the following list of compounds may be cited:
- 2,3-didodecanoxy-7-carboxyphenazine (compound 1) ;
- 2,3-didodecanoxy-7,8-diaminophenazine (compound 2) ;
- 2,3-didodecanoxyphenazine (compound 3) ;
- 2,3-didodecanoxy-7-dimethylaminoethylcarbamoylphenazine (compound 4) ;
- 2-hydroxy-3-ethoxy-7-ethylcarboxyphenazine (compound 5) ;
or anyone of its pharmaceutically acceptable salts,
for use as an agent for treating cancer.

Still according to an other variant, a compound of the invention may be represented by a compound of formula (I) or (Ia) and also by the 2,7-dihydroxyphenazine, or anyone of its pharmaceutically acceptable salts, for use as an agent for treating pancreatic cancer.

According to a second aspect, a subject-matter of the present invention further relates to particular compounds of formula (I) such as defined above, wherein R₁, R₂, R₃ and R₄ are such as defined above,
provided that:
- when R₁ and R₂ both represent a hydroxyl group and R₄ represents a hydrogen atom, then R₃ is different from a chloride atom, a hydroxyl or a methoxy group;
- when R₁ and R₂ both represent a C₉-alkoxy or C₁₁- alkoxy group, then at least one of R₃ or R₄ is different from a hydrogen atom;
- when R₂, R₃ and R₄ represent a hydrogen atom, then R₁ is different from a hydroxyl group;
- R₁, R₂, R₃ and R₄ cannot all represent a hydrogen atom; and
- when R₁ and R₂ represent an amino group, then at least one of R₃ and R₄ is different from a hydrogen atom,
or one of its pharmaceutically acceptable salts.

In another preferred variant, a compound of the invention may be represented by a compound of formula (Ib) : 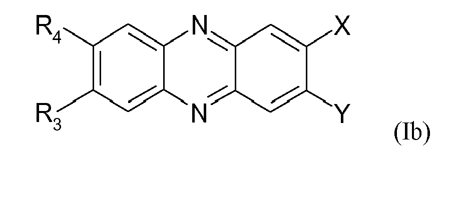 wherein R₃ and R₄ are such as define above, X and Y may represent, independently the one of the other, -OR₈ or -OCOR₉, with R₈ and R₉ being such as defined above,
provided that:
- when X and R₃ represent a hydroxyl group, then at least one of Y or R₄ is different from a hydrogen atom;
- when X and Y represent a hydroxyl group and R₄ represents a hydrogen atom, then R₃ is different from a chloride atom, a hydroxyl or a methoxy group; and
- when X and Y both represent a C₉-alkoxy or C₁₁-alkoxy group, then at least one of R₃ or R₄ is different from a hydrogen atom;
or one of its pharmaceutically acceptable salts.

Still according to this particular aspect, the following list of compounds may be cited:
- 2,3-didodecanoxy-7-carboxyphenazine (compound 1) ;
- 2,3-didodecanoxy-7,8-diaminophenazine (compound 2) ;
- 2,3-didodecanoxyphenazine (compound 3) ;
- 2,3-didodecanoxy-7-dimethylaminoethylcarbamoylphenazine (compound 4) ;
- 2-hydroxy-3-ethoxy-7-ethylcarboxyphenazine (compound 5) ;
- 2,3-diopalmitoylphenazine (compound 7) ;
- 2,3-dihydroxy-7,8-dichlorophenazine (compound 8) ;
- 2,3-didodecanoxy-7,8-ditosylaminophenazine (compound 9) ;
- 2,3-dihydroxy-7-nitrophenazine (compound 11) ;
- 2,3-dihydroxy-7,8-ditosylaminophenazine (compound 12) ;
- 2,3-dihydroxy-7,8-diamino(Boc)phenazine (compound 13) ;
- 2,3-diethoxy-7-ethylcarboxyphenazine (compound 14) ;
- 2,3-dihydroxy-7-carboxyphenazine (compound 17) ;
or one of its pharmaceutically acceptable salts.

According to another aspect of the invention, at least one of the particular compounds of the present invention may be used as a medicament.

Accordingly, the instant invention is also directed to a pharmaceutical composition containing as an active ingredient at least one of the particular compounds of the invention.

The compounds of the present invention can be prepared by conventional methods of synthesis practiced by those skilled in the art. In particular, the used synthesis implements purification by column and/or (re)crystallization, realized at atmospheric pressure. The general reaction sequences outlined below represent a general method useful for preparing the compounds of the present invention and are not meant to be limiting in scope or utility.

Thus, the compounds of general formula (I) can be prepared *via* one step sequence according to scheme 1 below.

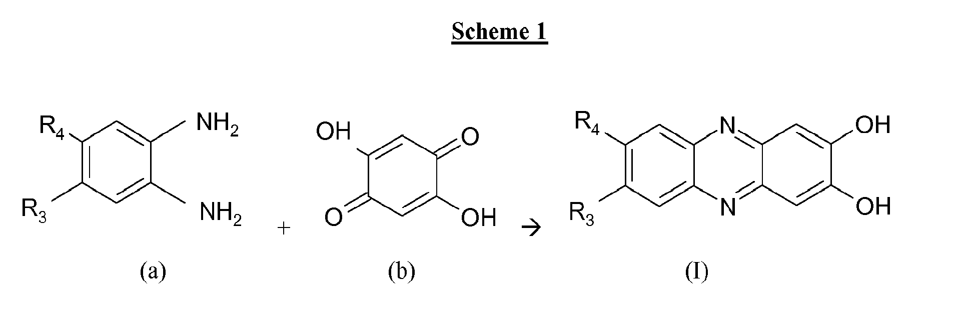

The general proceedings for the preparation of 2,3-dihydroxyphenazine is as follows:
A mixture of o-diamine benzene (or an analogue thereof) and 2,5-dihydroxy-*p-*benzoquinone, in water or absolute ethanol, is heated to reflux during 1h30 to 24h.

The completion of the reaction is controlled by CCM with ethyl acetate as eluent.

The reaction mixture is concentrated under reduced pressure.

The crude residue is washed with an organic solvent and filtered through sintered glass.

The preparation of derivatives of phenazine according to the present invention may consist, either in a simple modification of R₃ and/or R₄, and/or in an alkylation and/or esterification of at least one of the hydroxyl groups.

The starting compounds of formula (a) and (b) are commercially available or can be prepared according to methods known to the person skilled in the art.

The chemical structures of some compounds according to the invention are illustrated in the following Table I.

**Table I**

| 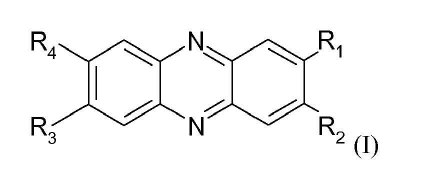 | | | | |
|---|---|---|---|---|
| N^{o} | **R1** | **R2** | **R3** | **R4** |
| **1** | OC₁₂H₂₅ | OC₁₂H₂₅ | CO₂H | H |
| **2** | OC₁₂H₂₅ | OC₁₂H₂₅ | NH₂ | NH₂ |
| **3** | OC₁₂H₂₅ | OC₁₂H₂₅ | H | H |
| **4** | OC₁₂H₂₅ | OC₁₂H₂₅ | 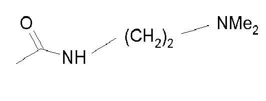 | H |
| **5** | OH | OCH₂CH₃ | CO₂CH₂CH₃ | H |
| **6** | H | H | H | H |
| **7** | 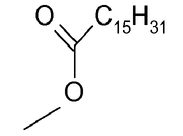 | 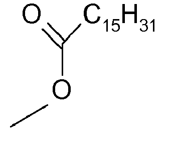 | H | H |
| **8** | OH | OH | Cl | C1 |
| **9** | OC₁₂H₂₅ | OC₁₂H₂₅ | 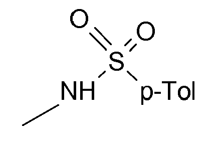 | 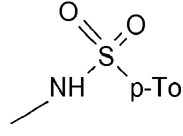 |
| **10** | NH₂ | NH₂ | H | H |
| **11** | OH | OH | NO₂ | H |
| **12** | OH | OH | 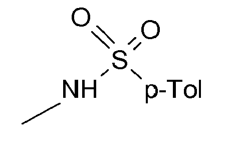 | 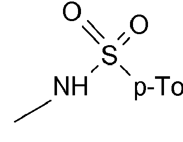 |
| **13** | OH | OH | NH-Boc* | NH-Boc* |
| **14** | OCH₂CH₃ | OCH₂CH₃ | CO₂CH₂CH₃ | H |
| **15** | OH | H | H | H |
| **16** | OH | OH | C1 | H |
| **17** | OH | OH | CO₂H | H |
| **18** | OH | OH | H | H |

| | | | | |
|---|---|---|---|---|
| * The term "Boc" means a tert-butyloxycarbonyl group. | | | | |

The following examples illustrate in detail the preparation of compounds according to the invention.

Starting compounds and reactants, unless otherwise indicated, are commercially available or described in literature, or can be prepared according to methods described in literature or known to one skilled in the art.

### EXAMPLES

Commercial analytical-grade reagents are obtained from commercial suppliers and used directly without further purification. The 1H and 13C NMR spectra are recorded in CDCl₃, acetone-*d₆* and DMSO-*d₆*, determined with a AC250 Brucker spectrometer operating at 250 MHz and referenced to the solvent. Chemical shifts are expressed in ppm and coupling constants (*J*) are in hertz. Splitting patterns are designed as s, singlet; br s, broad singlet; d, doublet; dd, doublet doublet; t, triplet; td, triplet doublet ; qt, quintuplet.

Elemental and MS analyses are performed by the Spectropole of Marseille. ESI mass spectral analyses are recorded with a 3200 QTRAP (Applied Biosystems SCIEX) mass spectrometer.

Preparative flash column chromatographies are performed using silica gel (Merck) G60 230-240 mesh.

### Example 1: preparation of compounds 12, 9 and 2 of the invention 1.1) 4,5-ditosylaminobenzene-1,2-diamine

4,5-ditosylaminobenzene-1,2-diamine is prepared according to *Eur. J. Org. Chem.,* 4, 2006: 947). To a solution of 1,2-dinitro-4,5-ditosylaminobenzene (1g, 1.97 mmol, 1 equiv.), prepared according to J. Chem. Soc., 1962: 1170, in dry methyl alcohol (30 mL) is added Pd/C 5%. The mixture is then stirred overnight at room temperature under hydrogen pressure (P = 40 bars), and extracted by methyl alcohol for 4hrs. The solvent is concentrated to afford 4,5-ditosylaminobenzene-1,2-diamine as an orange solid.

### 1.2) 2,3-dihydroxy-7,8-ditosylaminophenazine (compound 12)

The crude 4,5-ditosylaminobenzene-1,2-diamine (224 mg, 0.50 mmol, 1 equiv.) and 2,5-dihydroxy-1,4-benzoquinone (77.3 mg, 0.55 mmol, 1.1 equiv.) are mixed in refluxing ethyl alcohol for 24 hrs. After concentration of the reaction solvent, the crude product is taken up in acetonitrile. The insoluble suspension is isolated by filtration and washed by diethyl ether affording 2,3-dihydroxy-7,8-ditosylaminophenazine as a brown solid (quantitative yield). ¹H NMR (acetone-*d₆*) δ = 2.63 (s, 6H, CH₃), 7.22 (s, 2H, aromatic H), 7.35 (d, 4H, *Jₒᵣₜₕₒ =* 8,75 *Hz,* tosyl H), 7.69 (s, 2H, aromatic H), 7.76 (d, 4H, *Jₒᵣₜₕₒ* = 8,75 *Hz,* tosyl H), 8.45 (s, 2H, N*H*Ts). MS (ESI)⁺ : m/z = 551.2 [M+H]⁺

### 1.3) 2,3-didodecanoxy-7,8-ditosylaminophenazine (compound 9)

To a solution of 2,3-dihydroxy-7,8-ditosylaminophenazine (246.7 mg, 0.45 mmol, 1 equiv.) in dry DMF (10 mL) at room temperature is added, K₂CO₃ (618.4 mg, 4.48 mmol, 10 equiv.) and dropwise 1-bromododecane (230 µL, 0.95 mmol, 2.1 equiv.). The residue is taken up in water, neutralized by HCl solution (1M) and extracted with ethyl acetate. The organic layers are then concentrated and purified by SiO₂ chromatography (eluent : dichloromethane/methyl alcohol 10/0, 99/1, 98/2, 95/5) to give 2,3-didodecanoxy-7,8-ditosylaminophenazine as a red lacquer (38% yield). ¹H NMR (acetone-*d₆*) δ = 0.87 (m, 6H, CH₃), 1.29 (br s, 32H, CH₂), 1.60 (m, 4H, O-CH₂- CH₂-C*H₂* , 1.94 (m, 4H, O-CH₂-C*H₂*,, 2.37 (s, 6H, tosyl CH₃), 4.26 (t, 4H*, J₁* = 12.5 *Hz, J₂* = 6 *Hz,* O-CH₂), 7.24 (s, 2H, aromatic H), 7.36 (d, 4H, *Jₒᵣₜₕₒ =* 8 *Hz*, tosyl H), 7.70 (s, 2H, aromatic H), 7.75 (d, 4H, *Jₒᵣₜₕₒ =* 8 Hz, tosyl H). MS (ESI)⁺ : m/z = 887.4 [M+H]⁺ Calculated for C₅₀H₇₀O₆N₄S₂ : C 67.69, H 7.95, N 6.31 ; found : C 68.0, H 7.20, N 6.0.

### 1.4) 2,3-didodecanoxy-7,8-diaminophenazine (compound 2)

A solution of 2,3-didodecanoxy-7,8-ditosylaminophenazine (121.4 mg, 0.14 mmol, 1 equiv.) and H₂SO₄ (265 µL, 4.97 mmol, 36 equiv.) is stirred at room temperature for 1h30. Water (12 mL) and NaHCO₃ saturated solution (5 mL) are then added; the precipitate is filtered, and successively washed with NaHCO₃ saturated solution (until pH > 7) and CH₂Cl₂ to afford 2,3-didodecanoxy-7,8-diaminophenazine as a brown solid (49%). ¹H NMR (DMSO-*d₆*) δ = 0.84 (t, 6H, J = 6.75 *Hz,* CH₃), 1.23 (br s, 32H, CH₂), 1.49 (m, 4H, O-CH₂-CH₂-C*H₂*,, 1.78 (m, 4H, O-CH₂-C*H₂*, 4.10 (t, 4H, J= 6 *Hz,* O-CH₂), 5.87 (br s, 4H, NH₂), 6.86 (s, 2H, aromatic H), 7.17 (s, 2H, aromatic H). MS (ESI)⁺ : m/z = 579.3 [M+H]⁺. MS (ESI)⁻ : m/z = 577.4 [M-H]⁻. Calculated for C₃₆H₅₈O₂N₄ . 3 CH₂Cl₂ . 3 H₂O :C 52.77, H 7.95, N 6.31 ; found : C 52.67, H 8.05, N 6.59.

### Example 2: preparation of 2,3-diopalmitoylphenazine (compound 7) of the invention

To a solution of palmitic acid (483.8 mg, 1.89 mmol, 2 equiv.) and 4-DMAP (60 mg, 0.49 mmol, 0.5 equiv.) in dry dichloromethane (15 mL) is added, at room temperature and under nitrogen, DCC (389.4 mg, 1.89 mmol, 2 equiv.). After stirring for 15 min, a solution of 2,3-dihydroxy-7,8-ditosylaminophenazine (200 mg, 0.94 mmol, 1 equiv.) (15 mL), whose the preparation is above-mentioned, is added dropwise in dry dichloromethane. The mixture is allowed to stir overnight. DCU precipitate is filtered and washed by dichloromethane. The filtrate is concentrated and the residue is taken up in THF. Finally the resulting insoluble is washed with ethyl acetate affording 2,3-diopalmitoylphenazine as a pale yellow solid (99% yield). ¹H NMR (CDCl₃) δ = 0.87 (t, 6H, J= 7 *Hz,* CH₃), 1.3 (br s, 44H, CH₂), 1.75 (m, 8H, O=C-CH₂-C*H₂*-C*H₂*), 2.64 (t, 4H, J = 7.5 *Hz ;* O=C-CH₂), 8.06 (s, 2H, aromatic H), 8.22 (dd, 2H, *Jₒᵣₜₕₒ* = 6.5 *Hz, Jₘₑₜₐ* = 3.5 *Hz,* aromatic H), 8.85 (dd, 2H, *Jₒᵣₜₕₒ* = 6.5 *Hz, Jₘₑₜₐ =* 3.5 Hz, aromatic H). ¹³C NMR (CDCl₃) δ = 14.27 (CH₃), 22.84, 25.05, 25.72, 29.31, 29.47, 29.51, 29.65, 29.79, 29.81, 28.83, 29.86 (CH₂), 32.06 (O=C-CH₂-CH₂-*C*H₂), 34.06 (O=C-CH₂-*C*H₂), 34.33 (O=C-*C*H₂), 121.68, 129.66, 130.88 (aromatic CH), 141.98, 143.51 (aromatic C), 145.62 (aromatic C-O), 170.94 (C=O). MS (ESI)⁺ : m/z = 690.0 [M+H]⁺ Calculated for C₄₄H₆₈N₂O₄. 1/3 DCU : C 76.00, H 10.03, N 4.98 ; found : C 75.96, H 10.37, N 4.97.

### Example 3: preparation of compounds 1 and 4 of the invention 3.1) 2,3-didodecanoxy-7-carboxydodecyl ester phenazine

To a solution of 2,3-dihydroxy-7-carboxyphenazine *(compound 17)* (250 mg, 0.98 mmol, 1 equiv.), prepared according to Org. Lett., 10, 2008: 4013, in dry DMF (9 mL) is added at room temperature, K₂CO₃ (1.35 g, 9.77 mmol, 10 equiv.) and dropwise 1-bromododecane (1.4 mL, 5.79 mmol, 6 equiv.). The mixture is allowed to stir overnight at 90°C, then cooled at room temperature and DMF is evaporated under reduced pressure. The residue is taken up in water, neutralized by HCl solution (1M) and extracted with CH₂Cl₂. The organic layers are then concentrated and pentane is added to precipitate 2,3-didodecanoxy-7-carboxydodecyl ester phenazine which is filtrated and isolated as a brown solid.

### 3.2) 2,3-didodecanoxy-7-carboxyphenazine (compound 1)

To a solution of 2,3-didodecanoxy-7-carboxydodecyl ester phenazine (350 mg, 0.46 mmol, 1 equiv.) in a mixture of THF/H₂O 2.5/1 (50/20 mL) are added at room temperature NaOH (190.9 mg, 4.77 mmol, 10 equiv.). The mixture is refluxed overnight, cooled to room temperature, acidified to pH 1 with HCl (1M) and cooled at 4°C. The precipitate is isolated by filtration affording 2,3-didodecanoxy-7-carboxyphenazine as a green solid (83% yield). ¹H NMR (CDCl₃) δ = 0.88 (t, 6H, *J* = 6.5 *Hz,* CH₃), 1.27 (br s, 32H, CH₂), 1.56 (m, 4H, O-CH₂-CH₂-C*H₂*), 1.97 (qt, 4H, *J* = 6.75 *Hz,* O-CH₂-C*H₂*), 4.26 (t, 4H, *J* = 6.75 *Hz,* O-CH₂), 7.40 (s, 1H, aromatic H), 7.44 (s, 1H, aromatic H), 8.23 (d, 1H, *Jₒᵣₜₕₒ* = *9* Hz, aromatic H), 8.38 (dd, 1H, *Jₒᵣₜₕₒ* = *9 Hz, Jₘₑₜₐ* = 1.75 Hz, aromatic H), 9.03 (d, 1H, *Jₘₑₜₐ =* 1.75 Hz, aromatic H). MS (ESI)⁺ : m/z = 593.4 [M+H]⁺. MS (ESI)⁻ : m/z = 591.4 [M-H]⁻. Calculated for C₃₇H₅₆N₂O₄ . NaCl: C 71.44, H 9.07, N 4.50 ; found : C 71.39, H 9.75, N 4.35.

### 3.3) 2,3-didodecanoxy-7-dimethylaminoethylcarbamoylphenazine (compound 4)

A solution of 2,3-didodecanoxy-7-carboxyphenazine (100 mg, 0.17 mmol, 1 equiv.) in thionyl chloride (1 mL, 13.8 mmol, 81 equiv.) is heated at 50°C for 1hr. The mixture is cooled at room temperature and thionyl chloride removed by distillation. To the yellow residue are successively added dry chloroform (4 mL), 1-amino-4-dimethylaminethane (19 µL, 0.17 mmol, 1 equiv.) and triethylamine (67 µL, 0.48, 2.8 equiv.). The mixture is allowed to stir at room temperature for 2 hrs. Chloroform is added (4 mL) and the mixture is washed with a Na₂CO₃ solution (2M). The organic layers are concentrated and pentane is added to precipitate 2,3-didodecanoxy-7-dimethylaminoethylcarbamoylphenazine which is filtrated and isolated as a yellow solid (44% yield). ¹H NMR (CDCl₃) δ = 0.87 (t, 6H, *J* = 6.5 *Hz,* CH₂-C-*H₃*), 1.26 (br s, 32H, CH₂), 1.54 (m, 4H, O-CH₂-CH₂-C*H₂*), 1.96 (qt, 4H, J= 6.75 *Hz,* O-CH₂-C*H₂*), 2.33 (s, 6H, N-CH₃), 2.61 (t, 2H, *J* = 6 *Hz,* N-CH₂), 3.62 (td, 2H, *J* = 6 *Hz, J =* 5 *Hz,* NH-CH₂), 4.23 (t, 4H, *J* = 6.75 *Hz,* O-CH₂), 7.33 (s, 1H, aromatic H), 7.34 (s, 1H, aromatic H), 8.20 (d + dd, 2H, *J* = 6 *Hz, J =* 1.5 *Hz,* aromatic H), 8.52 (d, 1H, *J* = 1.5 *Hz,* aromatic H). MS (ESI)⁺: m/z = 663.4 [M+H]⁺. MS (ESI)⁻ : m/z = 661.5 [M-H]⁻. Calculated for C₄₁H₆₆N₄O₃ . 5/3 H₂O : C 71.06, H 10.08, N 8.08 ; found : C 71.03, H 10.14, N 7.49.

### Example 4

Some compounds of the previous examples have been the subject of tests which have demonstrated their cytotoxicity against cancerous pancreatic cells (MiaPaca cells).

### Materials and methods

Pancreatic chemoresitant cancer MiaPaCa-2 cells are cultured in DMEM medium (Gibco) supplemented with 10% fetal bovine serum (FBS). Cells are seeded at a densitiy of 30,000 cells per well in 48 well View Plate (Packard) in 250 µL of medium containing the same components as described above. Cells are allowed to attach overnight (O/N) and then culture medium is removed and replace with fresh media alone as control or containing different concentrations of each of tested compounds. Plates are further incubated at 37 °C and 5% CO₂ for 48 hours. The number of viable cells remaining after the appropriate treatment is determined by (3-(4,5-dimethylthiazol-2-yl)-2,5-diphenyltetrazolium bromide, MTT) colorimetric assay.

The results regarding the level of cytotoxicity for some compounds of the invention and further considered in examples above-cited against MiaPaca cells are illustrated in Table 2 hereafter.

**Table 2**

| **Ref** | **Molecule** | **Cytotoxicity** (en %) |
|---|---|---|
| **1** | 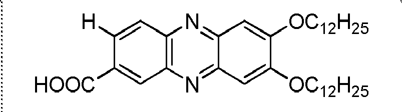 | X>50 |
| **2** | 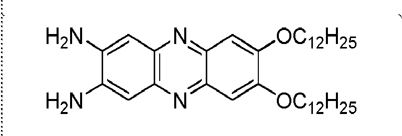 | X>50 |
| **3** | 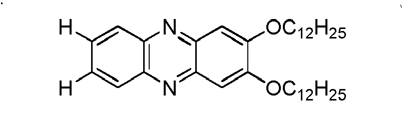 | X>50 |
| **4** | 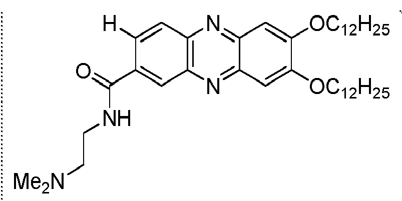 | X > 50 |
| **5** | 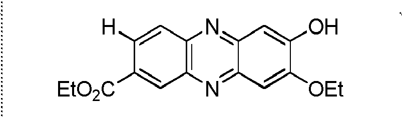 | X>50 |
| **6** | 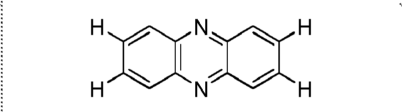 | 50 >X> 40 |
| **7** | 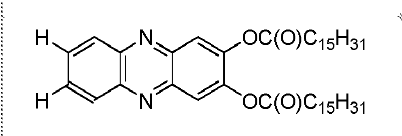 | 50 >X> 40 |
| **8** | 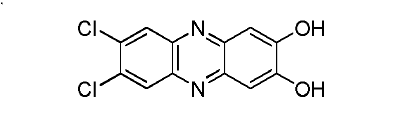 | 50 >X> 40 |
| **9** | 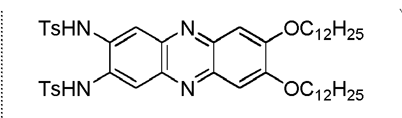 | 50 >X> 40 |
| **10** | 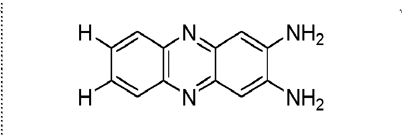 | 45 >X> 35 |
| **11** | 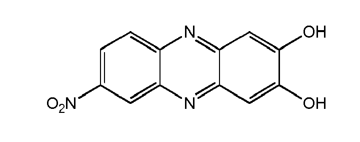 | 35 > X > 30 |
| **12** | 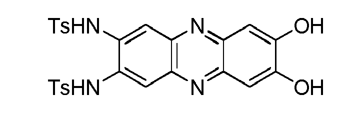 | 35 > X > 30 |
| **13** | 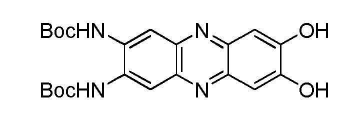 | 30 >X> 25 |
| **14** | 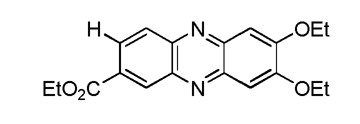 | 20 > X > 10 |
| **15** | 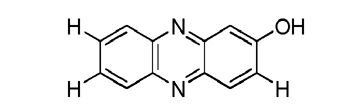 | 20>X>10 |
| **16** | 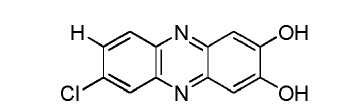 | 15>X>5 |
| **18** | 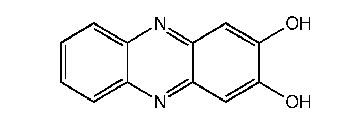 | 40>X>35 |

As discussed above, the present invention provides novel compounds having an effective antitumor and anti-cell proliferative activity.

Accordingly, said compounds are useful for the treatment of cancer, and more particularly for the treatment of pancreatic cancer.

The present invention is also related to a method for treating, preventing or avoiding cancer or solid tumour in a patient, comprising at least one step consisting in administering to said patient an effective amount of a compound of formula (I) and/or such as illustrated in examples 1 to 4 according to the present invention.

The present invention is also related to the use of a compound of formula (I) and/or such as illustrated in examples 1 to 4 or one of its pharmaceutically acceptable salts according to the present invention for the manufacture of a pharmaceutical composition intended for the treatment of cancer, and in particular of bladder cancer, breast cancer, ovarian cancer, pancreatic cancer, and gastric cancer, cervical cancer, colon cancer, endometrial cancer, head and neck cancer, lung cancer, melanoma, multiple myeloma, leukemia (e.g. myeloid, lymphocytic, myelocytic and lymphoblastic leukemias), non-hodgkin's lymphoma, prostate cancer, rectal cancer, malignant melanomas, and more particularly pancreatic cancer.

The present invention is also related to a compound of formula (I) and/or such as illustrated in examples 1 to 4 or one of its pharmaceutically acceptable salts according to the present invention for use as a medicament and more particularly for the treatment and/or the prevention of cancer or solid tumors, and more particularly pancreatic cancer.

The present invention is also directed to pharmaceutical compositions wherein these compositions comprise any one of the compounds as described herein, and optionally comprise a pharmaceutically acceptable carrier.

In certain preferred embodiments, these compositions optionally further comprise one or more additional therapeutic agents.

It will also be appreciated that certain of the compounds of present invention can exist in free form for treatment, or where appropriate, as a pharmaceutically acceptable derivative thereof.

According to the present invention, a pharmaceutically acceptable derivative includes, but is not limited to, pharmaceutically acceptable salts, esters, salts of such esters, or any other adduct or derivative which upon administration to a patient in need is capable of providing, directly or indirectly, a compound as otherwise described herein, or a metabolite or residue thereof, e.g., a prodrug.

"Pharmaceutically acceptable carrier", such as above-cited, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Fifteenth Edition, E. W. Martin (Mack Publishing Co., Easton, Pa., 1975) discloses various carriers used in formulating pharmaceutical compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutical composition, its use is contemplated to be within the scope of this invention. Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents.

Preservatives and antioxidants can also be present in the composition, according to the judgment of the formulator.

A compound according to the invention is preferably formulated in dosage unit form for ease of administration and uniformity of dosage. The expression "dosage unit form" as used herein refers to a physically discrete unit of anticancer agent appropriate for the patient to be treated. It will be understood, however, that the total daily usage of the compounds and compositions of the present invention will be decided by the attending physician within the scope of sound medical judgment. The specific therapeutically effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

Furthermore, after formulation with an appropriate pharmaceutically acceptable carrier in a desired dosage, the pharmaceutical compositions according to the invention can be administered to humans and other animals orally, rectally, parenterally, intracisternally, intravaginally, intraperitoneally, topically (as by powders, ointments, or drops), bucally, as an oral or nasal spray, or the like depending on the severity of the disease or cancer being treated.

The active compounds according to the invention can also be in micro-encapsulated form, eventually with one or more excipients as noted above.

It will also be appreciated that the compounds and pharmaceutical compositions of the present invention can be employed in combination therapies, that is, the compounds and pharmaceutical compositions can be administered concurrently with, prior to, or subsequent to, one or more other desired therapeutics or medical procedures.

## Claims

1. A compound of formula (I) : 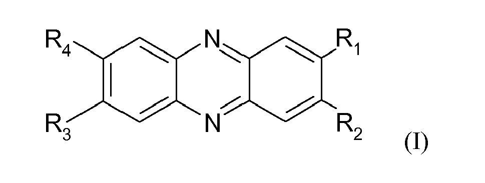 wherein R₁, R₂, R₃ and R₄, independently the ones of the others, represent :
- a hydrogen atom ;
- a halogen atom chosen among chlorine, fluorine, bromine and iodine atom;
- a cyano group;
- -NO2;
- -N(R₅)(R₆), with R₅ and R₆ may represent, independently the one of the other, a hydrogen atom, an alkyl group, linear or branched, in C₁-C₂₀, a benzyl group or - COR₁₀;
- an amido group ;
- -NHSO₂(R₇), with R₇ may represent an alkyl group, linear or branched, in C₁-C₂₀ or an aryl group;
- -OR₈;
- -OCOR₉, with R₈ and R₉ may represent a hydrogen atom or an alkyl group, linear or branched, in C₁-C₂₀;
- an alkyl or alkylthio group, linear or branched, satured or non satured, in C₁-C₂₀ ;
- -COR₁₀ with R₁₀ may represent a hydroxyl group or an alkoxy group, linear or branched, in C₁-C₂₀; and
- -CO-[NH-(CH₂)ₙ]ₘ-N(R₅)(R₆), -HN-CO-[(CH₂)ₙ-N(R₁₁)]ₚ-(CH₂)_{n'}-N(R₁₁)(R₆), or -O-CO-[(CH₂)ₙ-N(R₁₁)]p-(CH₂)_{n'}-N(R₁₁)(R₆), with
- R₅ and R₆ being are such as defined above,
- R₁₁ representing a hydrogen atom or a tert-butyloxycarbonyl group,
- p representing an integer ranging from 0 to 1, and
- n, n' and m, independently, representing an integer with n and n', independently, ranging from 2 to 6 and m ranging from 1 to 2, and, when m is 2, each n value may be identical or different one from another,
or anyone of its pharmaceutically acceptable salts,
provided that when R₁ and R₃ represent a hydroxyl group, then at least one of R₂ or R₄ is different from a hydrogen,
for use as an agent for treating cancer.

2. A compound of formula (I) according to claim 1, wherein R₁=R₂ and/or R₃=R₄, with R₁ to R₄ being such as defined in claim 1, for use as an agent for treating cancer.

3. A compound of formula (I) according to anyone of the preceding claims, wherein R₁=R₂ and represent -OR₈ or -OCOR₉, with R₈ and R₉ being such as defined in claim 1, for use as an agent for treating cancer.

4. A compound according to anyone of the preceding claims, wherein at least R₁, R₂ and R₃ are different from a hydrogen atom, for use as an agent for treating cancer.

5. A compound of formula (I) according to anyone of the preceding claims, defined by a formula (Ia): 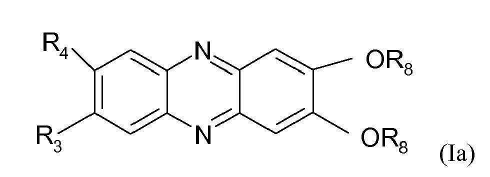 wherein R₃ and R₄ are such as defined in claims 1 or 2 and R₈ represent an alkyl group, linear or branched, in C₁-C₂₀, or anyone of its pharmaceutically acceptable salts, for use as an agent for treating cancer.

6. A compound according to anyone of the preceding claims, wherein R₃=R₄ and represent:
- a hydrogen atom;
- -N(R₅)(R₆);
- -NHSO₂(R₇); and
- a halogen atom, in particular chlorine,
with R₅, R₆ and R₇ being such as defined in claim 1, for use as an agent for treating cancer.

7. A compound according to anyone of the preceding claims, wherein R₃=R₄ and represent a hydrogen atom or NH₂, for use as an agent for treating cancer.

8. A compound according to anyone of claims 1 to 4, wherein R₃ represents - COR₁₀, with R₁₀ being such as defined in claim 1, for use as an agent for treating cancer.

9. A compound according to anyone of the preceding claims, wherein said compound is chosen among:
- 2,3-didodecanoxy-7-carboxyphenazine ;
- 2,3-didodecanoxy-7,8-diaminophenazine ;
- 2,3-didodecanoxyphenazine ;
- 2,3-didodecanoxy-7-dimethylaminoethylcarbamoylphenazine ;
- 2-hydroxy-3-ethoxy-7-ethylcarboxyphenazine ;
- phenazine ;
- 2,3-diopalmitoylphenazine ;
- 2,3-dihydroxy-7,8-dichlorophenazine ;
- 2,3-didodecanoxy-7,8-ditosylaminophenazine ;
- 2,3-diaminophenazine;
- 2,3-dihydroxy-7-nitrophenazine ;
- 2,3-dihydroxy-7,8-ditosylaminophenazine ;
- 2,3-dihydroxy-7,8-diamino(Boc)phenazine ;
- 2,3-diethoxy-7-ethylcarboxyphenazine ;
- 2-hydroxyphenazine;
- 2,3-dihydroxy-7-chlorophenazine ;
- 2,3-dihydroxy-7-carboxyphenazine ;
or anyone of its pharmaceutically acceptable salts, for use as an agent for treating cancer.

10. A compound according to anyone of the preceding claims and the 2,7-dihydroxyphenazine, or anyone of its pharmaceutically acceptable salts, for use as an agent for treating pancreatic cancer.

11. A Compound of formula (I) such as defined in anyone of the claims 1 to 4, wherein R₁, R₂, R₃ and R₄ are such as defined in anyone of the preceding claims,
provided that:
- when R₁ and R₂ both represent a hydroxyl group and R₄ represents a hydrogen atom, then R₃ is different from a chloride atom, a hydroxyl or a methoxy group;
- when R₁ and R₂ both represent a C₉-alkoxy or C₁₁-alkoxy group, then at least one of R₃ or R₄ is different from a hydrogen atom;
- when R₂, R₃ and R₄ represent a hydrogen atom, then R₁ is different from a hydroxyl group;
- R₁, R₂, R₃ and R₄ cannot all represent a hydrogen atom; and
- when R₁ and R₂ represent an amino group, then at least one of R₃ and R₄ is different from a hydrogen atom,
or one of its pharmaceutically acceptable salts.

12. Compound according to the preceding claim, wherein said compound is chosen among:
- 2,3-didodecanoxy-7-carboxyphenazine ;
- 2,3-didodecanoxy-7,8-diaminophenazine ;
- 2,3-didodecanoxyphenazine ;
- 2,3-didodecanoxy-7-dimethylaminoethylcarbamoylphenazine ;
- 2-hydroxy-3-ethoxy-7-ethylcarboxyphenazine ;
- 2,3-diopalmitoylphenazine ;
- 2,3-dihydroxy-7,8-dichlorophenazine ;
- 2,3-didodecanoxy-7,8-ditosylaminophenazine ;
- 2,3-dihydroxy-7-nitrophenazine ;
- 2,3-dihydroxy-7,8-ditosylaminophenazine ;
- 2,3-dihydroxy-7,8-diamino(Boc)phenazine ;
- 2,3-diethoxy-7-ethylcarboxyphenazine ;
- 2,3-dihydroxy-7-carboxyphenazine ;
or one of its pharmaceutically acceptable salts.

13. Compound according to claim 11 or 12, for use as a medicament.

14. Pharmaceutical composition containing as an active ingredient at least one compound according to claim 11 or 12.
